(19) Europäisches Patentamt
European Patent Office
Office européen des brevets

(11) Veröffentlichungsnummer: **0 240 601**
**A1**

(12) **EUROPÄISCHE PATENTANMELDUNG**

(21) Anmeldenummer: **86115436.7**

(22) Anmeldetag: **07.11.86**

(51) Int. Cl.⁴: **C07C 43/13**, C07C 43/11, C07C 43/12, C07C 143/74, D06M 13/18, D06M 13/40

(30) Priorität: **04.04.86 DE 3611302**

(43) Veröffentlichungstag der Anmeldung: **14.10.87 Patentblatt 87/42**

(84) Benannte Vertragsstaaten: **AT BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Wehowsky, Frank, Dr.**
**Talhauser Strasse 27**
**D-8269 Burgkirchen(DE)**

(54) **Fluorierte, mehrwehtige Alkohole, Verfahren zu ihrer Herstellung und ihre Verwendung.**

(57) Es wird ein Verfahren zur Herstellung mehrwertiger, fluorierter Alkohole beschrieben, bei dem Anlagerungsprodukte von Epichlorhydrin an Perfluoralkyl-Gruppen enthaltende Alkohole zunächst mit einem Alkali-, Ammonium-oder Erdalkalisalz einer aliphatischen Carbonsäure mit 1 bis 6 C-Atomen umgesetzt werden. Die entstandenen Carbonsäureester werden anschließend mit starker Säure oder starkem Alkali verseift. Durch das neue Verfahren werden mehrwertige Alkohole mit einer Perfluoralkylgruppe zur Verfügung gestellt, die ein besonders günstiges Verhältnis von reaktionsfähigeren primären zu weniger gut reagierenden sekundären OH-Gruppen aufweisen. Diese Verbindungen können als stark oberflächenaktive Stoffe sowie als Ausgangsstoff für die Herstellung von Verbindungen für die öl-und schmutzabweisende Ausrüstung von Textilien, Leder, Holz oder Papier verwendet werden. Gegenstand der Erfindung sind auch neue mehrwertige Alkohole, die eine Perfluoralkylethoxy-Gruppe aufweisen.

EP 0 240 601 A1

**Fluorierte, mehrwertige Alkohole, Verfahren zu ihrer Herstellung und ihre Verwendung**

Die Erfindung betrifft ein Verfahren zur Herstellung mehrwertiger, fluorierter Alkohole gemäß Anspruch 1, deren Verwendung und mehrwertige, fluorierte Alkohole gemäß Anspruch 10.

In der US-PS 3 872 058 werden unter anderem fluorierte Hydroxylverbindungen der Formeln
$R_f(CH_2)_3OCH_2CHOHCH_2OH$ und
$R_f(CH_2)_2SC_3H_6OCH_2CHOHCH_2OH$
beschrieben, in denen $R_f$ eine Perfluoralkylgruppe mit 4 bis 16 C-Atomen bedeutet. Gemäß den Beispielen 7 und 8 werden diese Verbindungen durch Umsetzung von Perfluoralkyliodid bzw. Perfluoralkylethanthiol mit 1-Allyloxy-2,3-dihydroxy-propan in Gegenwart von Azodiisobutyronitril hergestellt. Sofern Perfluoralkyliodid eingesetzt wurde, entsteht zunächst eine iodhaltige Anlagerungsverbindung, die anschließend katalytisch hydriert werden muß. Die angegebenen Herstellverfahren sind umständlich, kostenaufwendig und ergeben ungünstige Ausbeuten.

Aus GB-PS 1 524 631 ist es bekannt, 1,1,2,2,3,3,4,4-Octafluor-pentanol mit 2,3-Epoxy-1-propanol (Glycid) in Gegenwart von Alkalihydroxid umzusetzen, wobei Verbindungen der Formel
$H(CF_2CF_2)_2CH_2O(CH_2CHOHCH_2O)_rH$,
in der r = 1 bis 50 ist, entstehen. Wegen der Neigung des 2,3-Epoxy-1-propanols zu Explosionen, muß die Reaktion unter besonderen Sicherheitsvorkehrungen durchgeführt werden. Es entstehen hierbei bevorzugt (nach den Angaben in der genannten Literaturstelle sogar ausschließlich) Polyole, die neben einer Vielzahl von sekundären OH-Gruppen nur eine primäre OH-Gruppe im Molekül aufweisen. Solche Polyole sind für weitere Umsetzungen an der OH-Gruppe weniger geeignet, da sekundäre OH-Gruppen nicht so leicht und glatt reagieren wie primäre.

Ähnlich dem vorangehend beschriebenen Verfahren werden gemäß US-PS 3,470,258 mit Perfluoralkyl-Gruppen substituierte Methanole mit 2,3-Epoxy-1-propanol in Gegenwart von tertiären Aminen wie Triethylamin oder Pyridin umgesetzt und hierbei ein Gemisch von drei Arten von Verbindungen erhalten:

1.) solche mit ausschließlich gerader Polyglycerin-Kette (wie in GB-PS 1 524 631 beschrieben),
2.) solche mit ausschließlich verzweigter Polyglycerin-Kette und
3.) solche, die in einem Molekül gerade und verzweigte Polyglycerin-Anteile aufweisen.

Die Nachteile dieses Verfahrens sind dieselben wie bei GB-PS 1 524 631 ausgeführt.

Es wurde nun ein Verfahren gefunden, nach dem es möglich ist, fluorierte, mehrwertige Alkohole herzustellen, die ausschließlich eine verzweigte Polyglycerin-Kette und damit ein Maximum an primären Alkoholgruppen aufweisen. Es handelt sich um ein Verfahren zur Herstellung von Verbindungen der Formel

$$R_f(Q)_k-A-O-(CH_2-\underset{\displaystyle CH_2OH}{\underset{|}{C}H}-O)_pH \qquad (I)$$

worin bedeuten:

$R_f$ einen Perfluoralkylrest der Formel $Z-(CF_2)_x-$, in der Z = H; F oder

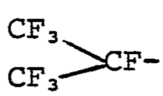

und x eine Zahl von 1 bis 20 ist, oder einen Perfluoralkoxyperfluoralkylrest;

Q eine der folgenden Gruppen: $-CO-$; $-(CH_2)_nCO-$; $-SO_2NR-$; $-(CH_2)_nSO_2NR-$; $-(CH_2)_nS-$; $-(CH_2)_nSO-$ oder $-(CH_2)_nSO_2-$, in denen R = H oder Alkyl-mit 1 bis 6 C-Atomen und n eine Zahl von 1 bis 4 ist,

k = Null oder 1,

A eine der folgenden Gruppen: $C_mH_{2m}-$;

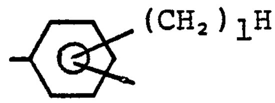 oder 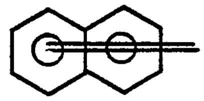

in denen m eine Zahl von 1 bis 6 und l = Null, 1 oder 2 ist,
p eine Zahl von 1 bis 15,
das dadurch gekennzeichnet ist, daß eine Verbindung der Formel

$$R_f-(Q)_k-A-O-(CH_2-\underset{CH_2X}{\underset{|}{C}}HO)_pH \qquad (II),$$

in der $R_f$; Q; k; A und p die oben angegebene Bedeutung haben und X = Cl oder Br ist, mit einem Alkali-, Ammonium-oder Erdalkalisalz einer aliphatischen Carbonsäure mit 1 bis 6 C-Atomen in einem polaren, aliphatischen Lösungsmittel bei 60 bis 200 °C unter normalem Atmosphärendruck oder erhöhtem Druck umgesetzt wird, nach Beendigung der Reaktion das Reaktionsgemisch mit einer starken Mineralsäure angesäuert oder mit einem Alkalihydroxid oder -carbonat versetzt, auf 40 bis 100 °C erwärmt wird und anschließend die unlöslichen Salze abgetrennt werden, dann das Lösungsmittel sowie etwa entstandene Carbonsäureester abdestilliert werden und, wenn erforderlich, die erzeugte Verbindung der Formel(I) weitergereinigt wird.

Die als Ausgangsprodukt verwendeten Verbindungen der Formel (II) sind bekannt. Sie können beispielsweise nach DE-OS 30 02 369 durch Anlagerung von 2,3-Epoxy-1-chlorpropan(Epichlorhydrin) an fluorierte Alkohle der Formel
$R_f$-(Q)$_k$-A-OH (III),
in der $R_f$; Q, k und A die weiter oben angegebene Bedeutung haben, in Gegenwart einer Lewis-Säure, beispielsweise $BF_3$; $AlCl_3$; $SnCl_3$ oder $SbCl_5$, hergestellt werden. Analog kann 2,3-Epoxy-1-brompropan eingesetzt werden. Auch in US-PS 4,377,710, Spalten 3 und 4, sowie in den Schriften US-PS 3 655 732, DE-OS 2 004 962, 2 034 155 und 2 151 035 ist die Herstellung von Verbindungen der Formel (II) beschrieben. Vorzugsweise werden wegen ihrer leichten Zugänglichkeit solche Verbindungen der Formel (II) eingesetzt, in denen X ein Chloratom bedeutet.

Das erfindungsgemäße Verfahren gibt besonders gute Ergebnisse, wenn Verbindungen der Formel (II) verwendet werden, in denen k = 0 ist. Ferner werden bevorzugt Verbindungen der Formel (II) eingesetzt, in denen A eine $-C_mH_{2m}$-Gruppe ist, da solche Verbindungen günstig hergestellt werden können. Das gleiche gilt für Verbindungen der Formel (II), in denen $R_f$ ein Perfluoralkylrest der Formel $Z-(CF_2)_x-$, in der x eine durch 2 ohne Rest teilbare Zahl ist.

Für das erfindungsgemäße Verfahren können Verbindungen der Formel (II) eingesetzt werden, in denen p eine Zahl von 1 bis 15 ist. Vorzugsweise werden solche Verbindungen verwendet, in denen p eine Zahl von 1 bis 10 und insbesondere solche, in denen p eine Zahl von 1 bis 5 bedeutet.

Die Verbindung der Formel (II), es können auch mehrere solcher Verbindungen sein, insbesondere solche, in denen p verschieden ist, wird in einem polaren, aliphatischen Lösungsmittel, gegebenenfalls unter Erwärmen, gelöst. Geeignete Lösungsmittel sind beispielsweise Glykol, Glykoldiether, beispielsweise Dimethyl-oder Diethylglykol, oligomere Glykole und deren Diether, beispielsweise Dimethyl-oder Diethyldiglykol, Glycerin und andere mehrwertige flüssige oder leicht schmelzbare Alkohole. Bevorzugt wird mindestens eine Verbindung der folgenden Formeln $C_qH_{2q+1}OH$ und $C_tH_{2t+1}O-(C_2H_4O)_uH$, worin bedeuten: q eine Zahl von 1 bis 4, t die Zahl 1 oder 2 und u eine Zahl von 1 bis 3, verwendet. Die Verbindung der Formel (II) braucht nicht vollständig gelöst zu werden, es kann ein mehr oder weniger großer ungelöster Rest verbleiben, der durch Bewegen der Lösung während der Reaktion, beispielsweise durch Rühren, dispergiert wird.

Diese Lösung bzw. Dispersion wird bei 60 bis 200 °C mit einem Alkali-, Ammonium-oder Erdalkalisalz einer aliphatischen Carbonsäure mit 1 bis 6 C-Atomen umgesetzt. Unter 60 °C verläuft die Reaktion im allgemeinen zu langsam, über 200 °C nimmt die Bildung unerwünschter Nebenprodukte stark zu.
Vorzugsweise wird in einem Temperaturbereich von 100 bis 170 °C unter dem autogenen Druck der Reaktionsmischung gearbeitet. Je nach gewählter Reaktionstemperatur und verwendetem Lösungsmittel können Drucke bis zu 3 MPa auftreten.

Bevorzugt werden die Natrium-, Kalium-, Ammonium-, Magnesium-oder Calciumsalze der aliphatischen Carbonsäuren eingesetzt. Besonders gute Ergebnisse werden erhalten, wenn Salze der Ameisen-, Essig- oder Propionsäure verwendet werden.

Je Chloratom in der Verbindung der Formel (II) wird mindestens 1 Molekül eines Alkali-oder Ammoniumsalzes bzw. 1/2 Molekül eines Erdalkalisalzes der Carbonsäure angewendet, das heißt, auf 1 mol der Verbindung der Formel (II), die p Chloratome enthält, nimmt man mindestens p mol eines Alkali-oder Ammoniumsalzes bzw. 0,5 p mol eines Erdalkalisalzes. Vorteilhaft wird ein Überschuß der Salze eingesetzt, das heißt, auf 1 mol der Verbindung der Formel (II) mit p Chloratomen 1,1 bis 3 p mol Alkali-oder Ammoniumsalz bzw. 0,55 bis 1,5 p mol Erdalkalisalz. Prinzipiell kann auch ein noch höherer Überschuß der carbonsauren Salze verwendet werden, doch wird hierdurch die Durchführung des Verfahrens unnötig erschwert, ohne einen deutlichen Nutzen zu erzielen.

Die Reaktion wird vorteilhaft in Gegenwart einer wirksamen katalytischen Menge eines im Reaktionsgemisch löslichen Iodids durchgeführt, da hierdurch eine deutliche Verkürzung der Reaktionsdauer und damit eine Verbesserung der Raum-Zeit-Ausbeute erzielt wird. Es werden zweckmäßig 0,05 bis 5 Gew.-%, bezogen auf die Verbindung der Formel (II), vom Iodid eingesetzt. Vorzugsweise werden 0,3 bis 3 Gew.-%, bezogen auf die Verbindung der Formel (II), verwendet. Geeignet sind eine Vielzahl Iodide, beispielsweise die in Wasser leicht löslichen Metalliodide. Vorzugsweise werden die Alkali-, Ammonium-oder Erdalkaliiodide eingesetzt, insbesondere Natrium-oder Kaliumiodid.

Die Reaktionsdauer hängt von den eingesetzten Substanzen und der gewählten Temperatur ab. Im allgemeinen ist die Umsetzung in 1 bis 60 h, häufig in 2 bis 24 h beendet.

Anschließend kann das Reaktionsgemisch auf zwei verschiedene Arten weiterbehandelt werden. In einer Ausführungsform wird die Reaktionsmischung mit einer starken Mineralsäure, beispielsweise Schwefelsäure, Phosphorsäure und insbesondere Chlorwasserstoff im Überschuß versetzt und während 0,5 bis 5 h auf einer Temperatur von 40 bis 100 °C gehalten. Unter 40 °C muß unnötig viel Zeit aufgewendet werden, über 100 °C verläuft die Reaktion zwar schnell, jedoch werden Produkte von schlechter Farbe erhalten. Vorzugsweise wird bei 60 bis 80 °C gearbeitet.

Nach Beendigung der zuletzt beschriebenen Reaktion wird der Säureüberschuß mit einem Alkali-, beispielsweise Natrium-, Kalium-oder Calciumhydroxid neutralisiert, die unlöslichen Salze,beispielsweise durch Filtrieren oder Zentrifugieren,abgetrennt und aus der verbliebenen Flüssigkeit das verwendete Lösungsmittel sowie etwa entstandene Carbonsäureester, gegebenenfalls unter Verwendung von Unterdruck, abdestilliert. Wurde eine flüchtige Mineralsäure, beispielsweise Chlorwasserstoff, verwendet, kann die Neutralisation entfallen, da die Säure bei der Destillation von dem gebildeten Reaktionsprodukt der Formel (I) abgetrennt wird. In einer weiteren Ausführungsform wird das Gemisch aus der Reaktion der Verbindung der Formel (II) mit dem carbonsauren Salz in Gegenwart des Iodids mit Alkali, beispielsweise einem Alkalihydroxid oder -carbonat im Überschuß versetzt und unter den gleichen Bedingungen wie bei dem mit Mineralsäure versetzten Produkt beschrieben, weiterverarbeitet.

Durch das erfindungsgemäße Verfahren werden Verbindungen der Formel (I) in guter bis sehr guter Ausbeute erhalten, die nach dem IR-Spektrum,der OH-Zahl sowie dem Cl-Gehalt eine hohe Reinheit aufweisen. Sie können in der Regel ohne weitere Reinigung als solche, beispielsweise als stark oberflächenaktive Tenside, verwen det werden. Auch als Ausgangsstoff für die Herstellung von Verbindungen für die öl-und schmutzabweisende Ausrüstung von Textilien, Leder, Holz oder Papier sind die nach dem neuen Verfahren erzeugten Verbindungen der Formel (I) in der Regel ohne weitere Reinigung anwendbar. Selbstverständlich können die erfindungsgemäß erhaltenen Verbindungen, sollte es in Einzelfällen erforderlich sein, nach üblichen Methoden, wie zum Beispiel Lösen in geeignetem Lösungsmittel, Behandlung mit absorbierenden Stoffen, beispielsweise Aktivkohle, und Ausfällen durch geeeignete Mittel oder Abdestillieren der verwendeten Lösungsmittel weitergereinigt werden.

Die Umsetzungen zur Gewinnung der Verbindungen der Formel (I) werden zweckmäßig unter Bewegung der Reaktionsmischung, beispielsweise durch Rühren oder Umschaufeln in einem rotierenden Behälter, durchgeführt. Eine Verwendung von Schutzgas, beispielsweise Stickstoff, ist nicht unbedingt erforderlich, jedoch in vielen Fällen vorteilhaft.

Gegenstand der Erfindung sind ferner neue Verbindungen der Formel

$$R_f\text{-}C_2H_4\text{-}O\text{-}(CH_2\text{-}\underset{\displaystyle CH_2OH}{\underset{|}{C}}HO)_pH \qquad (IV),$$

in der bedeuten:

$R_f$ einen Perfluoralkylrest der Formel $Z\text{-}(CF_2)_x\text{-}$, in der

Z = H; F oder

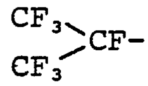

und x eine Zahl von 1 bis 20 ist, oder einen Perfluoralkoxyperfluoralkylrest und p eine Zahl von 1 bis 15.

Bevorzugt sind solche Verbindungen, in denen $R_f$ ein Perfluoralkylrest der Formel $Z$-$(CF_2)_x$-ist, in der Z = H;
F oder

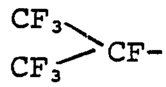

sein kann und x eine durch 2 ohne Rest teilbare Zahl von 2 bis 14 ist.

Als Ausgangsprodukte für die beanspruchten Verbindungen dienen Perfluoralkylethanole der Formel $R_fC_2H_4OH$, in der $R_f$ die vorstehend genannte Bedeutung hat. Solche Verbindungen sind teilweise am Markt erhältlich und können beispielsweise nach US-PS 3,824,296; 3,875,206 oder 4,219,681 hergestellt werden.

Diese Perfluoralkylethanole werden, wie oben bereits ausgeführt, beispielsweise nach den in der DE-OS 30 02 369, Seite 9 und Beispiel 2, sowie in der US-PS 4,377,710, Seiten 3 und 4 sowie Beispiel 1,beschriebenen Verfahren mit 2,3-Epoxy-1-chlor-propan oder 2,3-Epoxy-1-brom-propan in Gegenwart einer Lewis-Säure als Katalysator zu Verbindungen der Formel

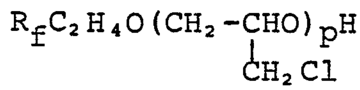

in der $R_f$ und p die weiter oben angegebene Bedeutung haben umgesetzt und anschließend, beispielsweise nach den weiter oben beschriebenen Verfahren, in die Verbindungen der Formel

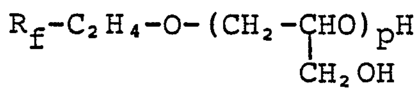
(IV)

übergeführt.

Die neuen Verbindungen sind wertvolle Ausgangsprodukte zur Herstellung von Verbindungen für die öl- und schmutzabweisende Ausrüstung von Textilien, Leder, Holz oder Papier. Sie können ferner als oberflächenaktive Tenside verwendet werden.

Nachfolgende Beispiele sollen die Erfindung näher erläutern:

Zunächst wird beispielhaft die Herstellung einiger Ausgangsstoffe beschrieben, die nachfolgend zum Unterschied von den erfindungsgemäßen Beispielen mit römischen Zahlen gekennzeichnet sind:

Ausgangsstoff I

In einem 1 $dm^3$ fassenden Kolben, der mit Rührer, Rückflußkühler, Thermometer sowie Tropftrichter ausgerüstet ist und sich in einem heizbaren Bad befindet, werden 200 g (0,76 mol) der Verbindung $C_4H_9C_2H_4OH$ mit 200 g Trifluor-trichlorethan verdünnt und mit 1 g $BF_3 \bullet Et_2O$ (0,5 Gew.-% bezogen auf $C_4H_9C_2H_4OH$) versetzt. Zu dieser Lösung werden 84,1 g (0,91 mol) Epichlorhydrin in 84 g Trifluor-trichlorethan gelöst, langsam zugetropft, wobei die Temperatur auf 50 °C steigt. Es wird 2 h bei der Siedetemperatur des Trifluor-trichlorethans nachgerührt und anschließend zunächst das Trifluor-trichlorethan bei leichtem Unterdruck, danach die nicht-umgesetzte Verbindung der Formel $C_4H_9C_2H_4OH$ und weitere flüchtige Nebenprodukte bei einem Druck von 1,86 kPa bis zu einer Kopftemperatur von 80 °C abdestilliert. Es werden so 214,6 g einer gelblichen Flüssigkeit erhalten.

Ausgangsstoffe II bis V

Analog der weiter oben angegebenen Vorschrift für Ausgangsstoff I werden verschiedene Perfluoralkylethanole sowie zwei Gemische verschiedener Perfluoralkylethanole mit Epichlorhydrin in unterschiedlichen Molverhältnissen umgesetzt. Das zur Herstellung von Ausgangsstoff IV verwendete Perfluoralkylethanol-Gemisch hat ein durchschnittliches Molekulargewicht entsprechend einer Verbindung der Formel $C_vF_{2v+1}C_2H_4OH$, in der v = 8,15 ist; das zur Herstellung für Ausgangsstoff V verwendete Gemisch hat ein Molekulargewicht entsprechend der gleichen Formel, in der v = 9,3 ist. Nach Abdestillation des Lösungsmittels und nicht-umgesetzter Ausgangsstoffe sowie flüchtiger Nebenprodukte werden jeweils gelbliche Flüssigkeiten oder wachsartige Verbindungen erhalten.

In nachfolgender Tabelle sind die Herstellbedingungen für für die Ausgangsstoffe I bis V zusammengefaßt:

TABELLE 1

| Herzustellender Ausgangsstoff | $C_vF_{2v+1}C_2H_4OH$ v = | Molverhältnis Epichlorhydrin/ $C_vF_{2v+1}C_2H_4OH$ | Endbedingungen der Destillation Druck Pa | Kopftemperatur °C |
|---|---|---|---|---|
| I | 4 | 1,2 : 1 | 1860 | 80 |
| II | 6 | 2 : 1 | 13 | 73 |
| III | 8 | 1,2 : 1 | 135 | 95 |
| IV | 6-14 (Ø = 8,15) | 1,2 : 1 | - | - |
| V | 8-16 (Ø = 9,3) | 2,0 : 1 | - | - |

Ausgangsstoff VI

In einem 2 $dm^3$ fassenden Kolben, der mit Rührer, Rückflußkühler, Tropftrichter sowie Thermometer ausgerüstet ist und sich in einem heizbaren Bad befindet, werden 500 g (1,06 mol) der Verbindung $C_vF_{2v+1}C_2H_4OH$, in der v die Werte 6 bis 14 mit einem Durchschnittswert von 8,15 bedeutet, mit 100 g Trifluor-trichlorethan verdünnt, mit 3,0 g $BF_3 \bullet Et_2O$ versetzt und unter Rühren aufgeheizt. Zu dieser Lösung werden während 6,5 h 982 g (10,6 mol) Epichlorhydrin langsam zugetropft, wobei eine exotherme Reaktion erfolgt. Die Vollständigkeit dieser Reaktion wird anhand der Wärmeentwicklung durch stündliche Nachdosierung von je 0,5 g $BF_3 \bullet Et_2O$ überprüft. Es werden so während 6 h bei 50 °C insgesamt 4,5 g $BF_3 \bullet Et_2O$ nachdosiert. Anschließend wird das Trifluor-trichlorethan bei einem Druck von 1,86 kPa bis zu einer Kopftemperatur von 90 °C abdestilliert. Es werden 1469 g einer braunen, pastösen Masse erhalten, die nach gaschromatographischer Untersuchung keinen nicht-umgesetzten Alkohol mehr enthält.

Ausgangsstoff VII

In einem 500 $cm^3$ fassenden Kolben, der mit Rührer, Rückflußkühler, Tropftrichter sowie Thermometer ausgerüstet ist und sich in einem Heizbad befindet, werden 57,1 g (0,1 mol) der Verbindung $C_8F_{17}SO_2N(C_2H_5)C_2H_4OH$ in 200 $cm^3$ Trifluor-trichlorethan gelöst, mit 0,5 g $BF_3 \bullet Et_2O$ versetzt und unter Rühren auf 47 °C erwärmt. Zu dieser Lösung werden 18,5 g (0,2 mol) Epichlorhydrin in 50 $cm^3$ Trifluor-trichlorethan gelöst zudosiert, wobei die Temperatur auf 50 °C steigt. Es wird noch während 2 h bei 49 °C nachgerührt, dann werden 0,2 g $BF_3 \bullet Et_2O$ nachdosiert und weitere 3 h auf 49 °C unter Rühren gehalten. Anschließend wird das Trifluor-trichlorethan bei leichtem Unterdruck abdestilliert. Es werden 73,0 g einer orangen, zähen Flüssigkeit erhalten.

Erfindungsgemäße Beispiele

Beispiel 1

In einem 2 $dm^3$ fassenden Kolben, der mit Rührer, Thermometer sowie Rückflußkühler ausgestattet ist und sich in einem heizbaren Bad befindet, werden 881 g (2,35 Val Cl) des Ausgangsstoffes III mit 280 g (2,85 mol) Kaliumacetat und 8,8 g (1 Gew.-%, bezogen auf Ausgangsstoff III) Kaliumiodid in 880 g Methyldiglykol gelöst und 8 h bei Normaldruck auf 140 °C erhitzt. Anschließend wird das Lösungsmittel im Vakuum abdestilliert und der Rückstand in 880 g Methanol gelöst. n diese Lösung wird bis zur Sättigung Chlorwasserstoff eingegast und anschließend 3 h unter Rückfluß erhitzt. Die ausgefallenen Salze werden abfiltriert und das Methanol abdestilliert. Es werden 780 g eines bräunlichen Produktes erhalten, von dem der Cl-Gehalt sowie die OH-Zahl (angegeben in mgKOH je 1000 mg der untersuchten Verbindung) ermittelt werden. In Tabelle 2 sind die Herstellbedingungen sowie die am Endprodukt ermittelten Werte angegeben. Nach der OH-Zahl hat das Produkt die Formel

$$C_8F_{17}-C_2H_4O[CH_2-\underset{CH_2OH}{CH}-O]_{1,64}H.$$

Beispiel 2

In einem Autoklaven werden 1394 g ( 3,71 Val Cl) Ausgangsstoff III mit 457 g (4,66 mol) Kaliumacetat und 14 g (1 Gew.-%, bezogen auf Ausgangsstoff III) Kaliumiodid in 1400 g Isopropanol gelöst. Der mit Stickstoff gespülte Autoklav wird 10 h auf 151 °C erhitzt, wobei ein Druck von 0,73 MPa gemessen wird. Das Reaktionsprodukt wird in einen 4 $dm^3$ fassenden Rührkolben überführt und die Lösung mit Chlorwasserstoff gesättigt. Die Temperatur steigt dabei von 25 auf 73 °C. Während 2,5 h wird bei 80 °C gerührt. Nach dem Abfiltrieren der ausgeschiedenen Salze wird das Isopropanol im Vakuum abdestilliert. Es werden 1306 g ocker gefärbtes Produkt erhalten, das im Infrarotsprektrum keine Ester-(1740 $cm^{-1}$) und Acetat-Bande (1650 $cm^{-1}$) zeigt. Die Säurezahl beträgt 0,7. In Tabelle 2 sind die Herstellbedingungen und die am Endprodukt ermittelte Ausbeute bzw. OH-Zahl und der Cl-Gehalt angegeben. Nach der OH-Zahl hat das Produkt die Formel

$$C_8F_{17}-C_2H_4O[CH_2-\underset{CH_2OH}{CH}-O]_{1,7}\ H.$$

Beispiele 3 bis 8

Wie in Beispiel 2 beschrieben, werden die Ausgangsstoffe I, II, IV und VI in unterschiedlichen Lösungsmitteln umgesetzt. Die Verseifung der intermediär gebildeten Ester wird ebenso wie in den vorangegangenen Beispielen anhand des Infrarotspektrums von Proben kontrolliert. Am Ende der Reaktion werden jeweils weder die Ester-noch die Acetat-Bande (siehe Beispiel 2) festgestellt. Die Herstellbedingungen sowie die am Endprodukt ermittelten Werte sind in Tabelle 2 angegeben. Nach der OH-Zahl sind bei den einzelnen Beispielen folgende Verbindungen gebildet worden

Beispiel 3: $C_4H_9-C_2H_4O-[CH_2-\underset{CH_2OH}{CH}-O]_{1,7}\ H$

Beispiel 4: $C_6F_{13}-C_2H_4-[CH_2-\underset{CH_2OH}{CH}-O]_{3,0}\ H$

Beispiel 5: $C_vF_{2v+1}-C_2H_4O-[CH_2-\underset{CH_2OH}{CH}-O]_{1,1}\ H;\quad v = 6\ \text{bis}\ 14$

Beispiel 6: $C_vF_{2v+1}-C_2H_4O-[CH_2-\underset{CH_2OH}{CH}-O]_{10,0}H;\quad v = 6\ \text{bis}\ 14$

Beispiel 7: $C_vF_{2v+1}-C_2H_4O-[CH_2-\underset{CH_2OH}{CH}-O]_{1,1}\ H;\quad v = 6\ \text{bis}\ 14$

Beispiel 8: $C_vF_{2v+1}-C_2H_4O-[CH_2-\underset{CH_2OH}{CH}-O]_{1,1}\ H;\quad v = 6\ \text{bis}\ 14.$

Beispiel 9

In einem Autoklaven von 250 $cm^3$ Inhalt werden 53 g (0,15 Val Cl) Ausgangsstoff V und 12,4 g (0,18 mol) Natriumformiat in 100 ml Methanol gelöst. Der mit Stickstoff gespülte Autoklav wird 60 h auf 170 °C erhitzt, dann abgekühlt und entspannt. Die Reaktionslösung wird in einen 500 $cm^3$ fassenden Rührkolben überführt und mit 100 $cm^3$ Methanol sowie 164 $cm^3$ 1-n-Natronlauge versetzt. Die Mischung wird 5 h auf 70 °C erhitzt. Nach Entfernung des Lösungsmittels durch Destillation (Enddruck 1,33 kPa; Endtemperatur 70 °C) wird der Rückstand in Trifluor-trichlorethan aufgenommen und von unlöslichen Anteilen abfiltriert. Nach Entfernung des Trifluor-trichlorethans werden 41,8 g einer gelben, festen Substanz erhalten. Wie in den vorangegangenen Beispielen enthält das Produkt nach der Verseifung im Infrarotspektrum keine Ester-oder Acetat-Bande mehr. Die Herstellbedingungen und am Endprodukt ermittelten Werte sind in nachfolgender Tabelle 2 angegeben. Nach der OH-Zahl hat die entstandene Verbindung die Formel

$$C_vF_{2v+1}-C_2H_4O-[CH_2-\underset{CH_2OH}{CH}-O]_{2,0}\ H;\quad v = 8\ \text{bis}\ 16.$$

Beispiel 10

In einem 250 $cm^3$ fassenden Autoklaven werden 40 g (0,1 Val Cl) Ausgangsstoff VII und 8,8 g (0,12 mol) Natriumformiat sowie 0,4 g Natriumiodid in 150 $cm^3$ Methanol gelöst. Der mit Stickstoff gespülte Autoklav wird 60 h auf 170 °C erhitzt. Nach Abkühlung und Entspannung wird die Reaktionsmischung in einem 1 $dm^3$ fassenden Rührkolben filtriert, mit 250 $cm^3$ Methanol verdünnt und mit Chlorwasserstoff gesättigt. Nun wird 5 h auf 62 °C erhitzt, anschließend abgekühlt, die ausgefallenen Salze abfiltriert und das Methanol aus dem Filtrat im Vakuum abdestilliert. Es werden 34,0 g einer gelbgrünen Substanz erhalten. Nach dem Infrarotspektrum kann im Endprodukt weder eine Ester-noch eine Formiat-Bande festgestellt werden. Die Herstellungsbedingungen und am Endprodukt ermittelten Werte sind in nachfolgender Tabelle 2 aufgeführt. Nach der OH-Zahl hat die entstandene Verbindung die Formel

$$C_8F_{17}SO_2N(C_2H_5)C_2H_4-O[CH_2-\underset{CH_2OH}{CH}-O]_{1,9}\ H.$$

TABELLE 2

| Beispiel Nr. | Ausgangs-stoff Nr. | Rest $C_vF_{2v+1}$ v= | Umsetzung mit Carbonsäuresalz Lösemittel | Dauer h | Temperatur °C | Endprodukt Ausbeute % d. Th. | OH-Zahl | Cl-Gehalt |
|---|---|---|---|---|---|---|---|---|
| 1 | III | 8 | MDG | 8 | 140 | 93 | 251 | 0,01 |
| 2 | III | 8 | IP | 10 | 151 | 98,5 | 252 | 0,01 |
| 3 | I | 4 | IP | 10 | 145 | 99 | 385 | < 0,01 |
| 4 | II | 6 | IP | 11,5 | 152 | 99,8 | 382 | 0,04 |
| 5 | IV | 6 - 14 (Ø=8,15) | IP | 9 | 144 | 94 | 215 | 0,06 |
| 6 | VI | 6 - 14 (Ø=8,15) | IP | 30 | 150 | 99,8 | 509 | 0,01 |
| 7 | IV | 6 - 14 (Ø=8,15) | M | 24 | 130 | 94 | 215 | 0,03 |
| 8 | IV | 6 - 14 (Ø=8,15) | E | 10 | 144 | 94 | 215 | 0,03 |
| 9 | V | 8 - 16 (Ø=9,3) | M | 60 | 170 | 85 | 240 | 0,1 |
| 10 | VII | 8 | M | 60 | 170 | 95 | 230 | 0,1 |

In Tabelle 2 bedeuten:
MDG = Methyldiglykol (Diglykolmonomethylether)
IP = Isopropylalkohol (Propan-2-ol)
M = Methanol
E = Ethanol

Ein Anwendungsgebiet der Verbindungen der Formel

$$R_f-C_2H_4-O-(CH_2-\underset{CH_2OH}{\underset{|}{C}}HO)_pH \quad (IV),$$

worin $R_f$ und p die auf Seite 8 unten angegebene Bedeutung haben, ergibt sich durch die sehr guten oberflächenaktiven Eigenschaften der genannten Verbindungen in Wasser.

In der nachfolgenden Tabelle sind die Oberflächenspannungen bei 20 °C von wäßrigen Lösungen der nach den Beispielen 4 bis 6 erzeugten Verbindungen in verschiedener Konzentration im Vergleich zu einem Perfluoralkyl-Gruppen enthaltenden Alkohol nach dem Stand der Technik angegeben. Der am reinen Wasser gemessene Wert beträgt 71 mN/m.

| Verbindung aus Beispiel | $R_f = C_vF_{2v+1}$ v = | p = | Oberflächenspannung in mN/m bei folgenden Konzentrationen in g/dm³ 1,0 | 0,3 | 0,1 |
|---|---|---|---|---|---|
| 4 | 6 | 3 | 19,7 | 20,2 | 22,2 |
| 5 | 6 - 14 | 1,1 | 18,9 | 19,2 | 19,7 |
| 6 | 6 - 14 | 10 | 21,0 | 22,6 | 24,6 |
| Z | 6 - 14 | - | 23,1 | 24,1 | 24,5 |

Verbindung Z = $C_vF_{2v+1}C_2H_4O(C_2H_4O)_{13}H$
worin bedeutet V = 6 bis 14.

Im nachfolgenden <u>Beispiel A</u> soll eine weitere Anwendungsmöglichkeit für Verbindungen der Formel IV dargestellt werden:

162,6 g (0,20 mol) Perfluoralkylethanol-Epichlorhydrin-Addukt mit dem Molverhältnis Perfluoralkylethanol zu Epichlorhydrin gleich 1 : 3 (das heißt y in der Formel 1 ist 3) als Addukt 1. Das Perfluoralkylethanol war ein im Handel erhältliches Gemisch mit Perfluoralkyl = $C_8F_{17}$ bis $C_{16}F_{33}$ (OH-Zahl = 69) und
57,0 g (0,10 mol) eines käuflichen Gemisches aus folgenden Isocyanat-Verbindungen

(α) 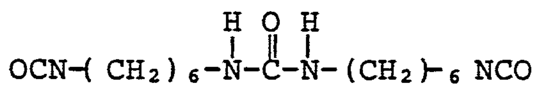

(β) 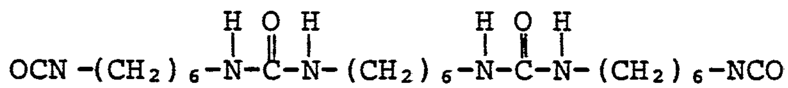

(γ) 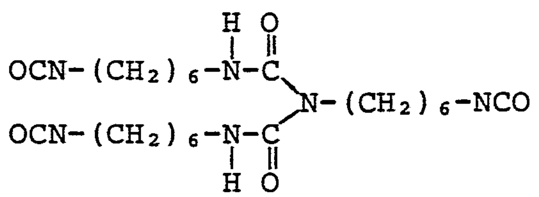 ,

das die Verbindung (γ) als Hauptbestandteil enthält,
werden in einem Kolben auf 110 °C erhitzt und bei dieser Temperatur 4 Stunden lang unter Rühren gehalten, worauf 4 Tropfen Dibutylzinndilaurat zugegeben und weitere 3 Stunden bei 110 °C gerührt werden. Zu dem erhaltenen wachsartigen, gelb gefärbten Perfluoralkylethanol-Epichlorhydrin-Isocyanat-Addukt werden
27,1 g (0,05 mol) einer erfindungsgemäßen Verbindung der Formel

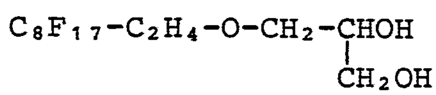

gelöst in 55 g Adipinsäure-di-n-butylether gegeben.
Das Molverhältnis der miteinander reagierenden Verbindungen beträgt 4 : 2 : 1.
Die Mischung wird 5 Stunden lang bei 110 °C unter Rühren gehalten und anschließend abgekühlt (2. Reaktionsstufe). Es wird ein wachsartiges, braun gefärbtes Produkt erhalten (Ausbeute 300 g, das sind 99,5 Gew.-% der Theorie). Die Bruttozusammensetzung der erhaltenen Verbindung entspricht der Formel

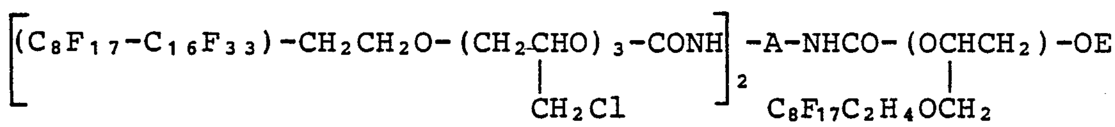

worin A bedeutet

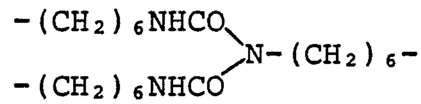

und E bedeutet

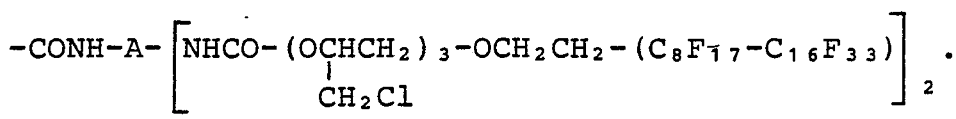

Die letztgenannte Verbindung wird mit Hilfe einer üblichen Spinnpräparation für Polyamidfasern getestet, die ca. 150 g von dieser Verbindung pro 1000 g Spinnpräparation enthält. Die Spinnpräparation besteht aus Wasser als Hauptkomponente, den üblichen ethoxylierten Fettalkoholen und langkettigen Aminoxiden als Präparationsmittel und ca. 15 Gew.-% der genannten Verbindung. Mit dieser Spinnpräparation wird ein Polyamid-6-Filament behandelt, um soviel von der Verbindung und dem Präparationsmittel auf das Filament aufzubringen, daß 0,05 Gew.-% Fluor und 1 Gew.-% Präparationsmittel auf dem Filament vorliegt, Gewichtsprozente bezogen auf das Gewicht des Filamentes. Dazu wird das Filament in üblicher Weise durch die Spinnpräparation gezogen, getrocknet und 30 Sekunden lang bei einer Temperatur von 200 °C gehalten (Wärmebehandlung, Kondensation). Aus dem so behandelten Filament wird ein Gewebe hergestellt. Auf dem Gewebe ist eine Fluorauflage von 0,05 Gew.-% und eine Präparationsmittelauflage von 1 Gew.-% vorhanden, Gewichtsprozente bezogen auf das Gewicht des Gewebes.

An dem Gewebe wird, wie weiter unten beschrieben, die Ölabweisung und die Wasserabweisung geprüft. Folgende Werte werden ermittelt:

| Ölabweisung | | Wasserabweisung | |
|---|---|---|---|
| nach der Konden-sation | nach der Koch-wäsche | nach der Konden-sation | nach der Koch-wäsche |
| 6 | 4 | 4 | 4 |

Zur Bestimmung des Ölabweisungswertes nach AATCC-Test 118 - 1966 werden drei Tropfen von einer bestimmten Testflüssigkeit (siehe unten) vorsichtig auf das zu prüfende Textilmaterial aufgelegt.

Einwirkungszeit: 30 Sekunden. Es wird der Wert angegeben, bei dem noch keine augenscheinliche Benetzung des Gewebes unter den Tropfen (nach abgelaufener Einwirkungszeit) hervorgerufen worden ist:

| Testflüssigkeit | Ölabweisungswert |
|---|---|
| Paraffinöl | 1 |
| Paraffinöl : n-Hexadecan = 65 : 35 | 2 |
| n-Hexadecan | 3 |
| n-Tetradecan | 4 |
| n-Dodecan | 5 |
| n-Decan | 6 |
| n-Octan | 7 |
| n-Heptan | 8 |

Ein Ölabweisungswert von 1 bedeutet den schlechtesten und ein Ölabweisungswert von 8 den besten Abweisungseffekt.

Zur Bestimmung des Wasserabweisungswertes nach DIN 53 888 - 1965 werden die zu prüfenden Textilien unter standardisierten Bedingungen beregnet, wobei gleichzeitig die Unterseite der Textilprobe mechanisch gerieben wird. Es wird der Wasserabperleffekt visuell mit den Noten 1 bis 5 beurteilt, wobei Note 1 den schlechtesten und Note 5 den besten Abperleffekt bedeutet.

Das Testergebnis zeigt, daß mit den erfindungsgemäßen Verbindungen der Formel (IV) Produkte erzeugt werden können, die eine sehr hohe Öl-und Wasserabweisung auf Textilien erreichen, was sie insbesondere für Textilbehandlungs-Präparationen geeignet macht.

## Ansprüche

1. Verfahren zur Herstellung von Verbindungen der Formel

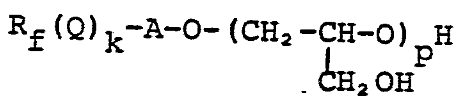

(I)

worin bedeuten:
$R_f$ einen Perfluoralkylrest der Formel $Z\text{-}(CF_2)_x\text{-}$
in der Z = H; F oder

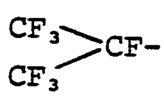

und x eine Zahl von 1 bis 20 ist, oder einen Perfluoralkoxyperfluoralkylrest;
Q eine der folgenden Gruppen $-CO-$; $-(CH_2)_nCO-$; $-SO_2NR-$; $-(CH_2)_nSO_2NR-$; $-(CH_2)_nS-$;$-(CH_2)_nSO-$oder $-(CH_2)_nSO_2-$,in denen R = H oder Alkyl-mit 1 bis 6 C-Atomen und n eine Zahl von 1 bis 4 ist,
k = Null oder 1,
A eine der folgenden Gruppen
dadurch gekennzeichnet, daß eine Verbindung der Formel

$-C_mH_{2m}-$; 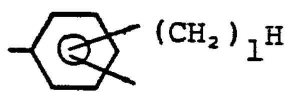 oder 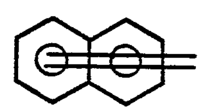

in denen m eine Zahl von 1 bis 6 und 1 = Null, 1 oder 2 ist,
p eine Zahl von 1 bis 15,
dadurch gekennzeichnet, daß eine Verbindung der Formel

$$R_f-(Q)_k-A-O-(\underset{\substack{|\\CH_2X}}{CH_2-CHO})_pH \qquad (II),$$

in der $R_f$; Q; k; A und p die oben angegebene Bedeutung haben und X = Cl oder Br ist, mit einem Alkali-, Ammonium-oder Erdalkalisalz einer aliphatischen Carbonsäure mit 1 bis 6 C-Atomen in einem polaren - (aliphatischen) Lösungsmittel bei 60 bis 200 °C unter normalem Atmosphärendruck oder erhöhtem Druck umgesetzt wird, nach Beendigung der Reaktion das Reaktionsgemisch mit einer starken Mineralsäure angesäuert oder mit einem Alkalihydroxid oder -carbonat versetzt, auf 40 bis 100 °C erwärmt wird und anschließend die unlöslichen Salze abgetrennt werden, dann das Lösungsmittel sowie etwa entstandene Carbonsäureester abdestilliert werden und, wenn erforderlich, die erzeugte Verbindung der Formel I weiter gereinigt wird.

2. Verfahren nach Anspruch 1, dadurch gekennzeichnet, daß ein Alkali-, Ammonium-oder Erdalkalisalz einer aliphatischen Monocarbonsäure mit 1 bis 3 C-Atomen eingesetzt wird.

3. Verfahren nach Anspurch 1 oder 2, dadurch gekennzeichnet, daß ein Natrium-, Kalium-, Ammonium-, Magnesium-oder Calciumsalz einer aliphatischen Carbonsäure eingesetzt wird.

4. Verfahren nach einem oder mehreren der Ansprüche 1 bis 3, dadurch gekennzeichnet, daß als polares (aliphatisches) Lösungsmittel mindestens eine Verbindung der folgenden Formeln
$C_qH_{2q+1}OH$ und $C_tH_{2t+1}O-(C_2H_4O)_uH$,
worin bedeuten: q eine Zahl von 1 bis 4, t die Zahl 1 oder 2 und u eine Zahl von 1 bis 3,
verwendet wird.

5. Verfahren nach einem oder mehreren der Ansprüche 1 bis 4, dadurch gekennzeichnet, daß die Umsetzung mit dem Alkali-, Ammonium-oder Erdalkalisalz einer aliphatischen Carbonsäure mit 1 bis 6 C-Atomen in Gegenwart einer wirksamen, katalytischen Menge mindestens eines im Reaktionsgemisch löslichen Iodides durchgeführt wird.

6. Verfahren nach Anspruch 5, dadurch gekennzeichnet, daß als Iodid Natrium-oder Kaliumiodid verwendet wird.

7. Verfahren nach einem oder mehreren der Ansprüche 1 bis 6, dadurch gekennzeichnet, daß eine Verbindung der Formel II eingesetzt wird, in der k = Null ist.

8. Verfahren nach einem oder mehreren der Ansprüche 1 bis 7, dadurch gekennzeichnet, daß eine Verbindung der Formel II eingesetzt wird, in der A eine $-C_mH_{2m}$-Gruppe ist.

9. Verfahren nach einem oder mehreren der Ansprüche 1 bis 8, dadurch gekennzeichnet, daß eine Verbindung der Formel II eingesetzt wird, in der p eine Zahl von 1 bis 10 ist.

10. Verfahren nach einem oder mehreren der Ansprüche 1 bis 9, dadurch gekennzeichnet, daß die Umsetzung bei 100 bis 170 °C durchgeführt wird.

11. Verbindungen der Formel

$$R_f-C_2H_4-O-(\underset{\substack{|\\CH_2OH}}{CH_2-CHO})_pH \quad ,$$

in der $R_f$ und p die im Anspruch 1 angegebene Bedeutung haben.

12. Verwendung der nach Anspruch 1 erzeugten Verbindungen der Formel als Ausgangsstoff für die Herstellung von Verbindungen für die öl-und schmutzabweisende Ausrüstung von Textilien, Leder, Holz oder Papier.

<u>Patentansprüche für Österreich:</u>

1. Verfahren zur Herstellung von Verbindungen der Formel

$$R_f(Q)_k-A-O-(CH_2-\underset{\substack{|\\CH_2OH}}{CH}-O)_pH \qquad (I)$$

worin bedeuten:
$R_f$ einen Perfluoralkylrest der Formel $Z-(CF_2)-_x$
in der Z = H; F oder

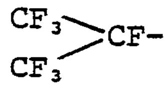

und x eine Zahl von 1 bis 20 ist, oder einen Perfluoralkoxyperfluoralkylrest;
Q eine der folgenden Gruppen -CO-; $-(CH_2)_nCO-$; $-SO_2NR-$; $-(CH_2)_nSO_2NR-$; $-(CH_2)_nS-$;$-(CH_2)$ $_nSO$-oder $-(CH_2)-$ $_nSO_2-$,in denen R = H oder Alkyl-mit 1 bis 6 C-Atomen und n eine Zahl von 1 bis 4 ist,
k = Null oder 1,
A eine der folgenden Gruppen

$-C_mH_{2m}-$; 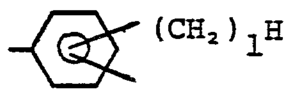 oder 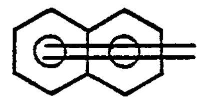

in denen m eine Zahl von 1 bis 6 und 1 = Null, 1 oder 2 ist,
p eine Zahl von 1 bis 15,
dadurch gekennzeichnet, daß eine Verbindung der Formel

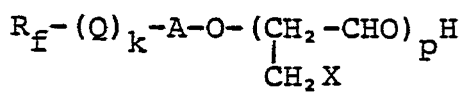 (II),

in der $R_f$; Q; k; A und p die oben angegebene Bedeutung haben und X = Cl oder Br ist, mit einem Alkali-, Ammonium-oder Erdalkalisalz einer aliphatischen Carbonsäure mit 1 bis 6 C-Atomen in einem polaren -(aliphatischen) Lösungsmittel bei 60 bis 200 °C unter normalem Atmosphärendruck oder erhöhtem Druck umgesetzt wird, nach Beendigung der Reaktion das Reaktionsgemisch mit einer starken Mineralsäure angesäuert oder mit einem Alkalihydroxid oder -carbonat versetzt, auf 40 bis 100 °C erwärmt wird und anschließend die unlöslichen Salze abgetrennt werden, dann das Lösungsmittel sowie etwa entstandene Carbonsäureester abdestilliert werden und, wenn erforderlich, die erzeugte Verbindung der Formel I weiter gereinigt wird.

2. Verfahren nach Anspruch 1, dadurch gekennzeichnet, daß ein Alkali-, Ammonium-oder Erdalkalisalz einer aliphatischen Monocarbonsäure mit 1 bis 3 C-Atomen eingesetzt wird.

3. Verfahren nach Anspurch 1 oder 2, dadurch gekennzeichnet, daß ein Natrium-, Kalium-, Ammonium-, Magnesium-oder Calciumsalz einer aliphatischen Carbonsäure eingesetzt wird.

4. Verfahren nach einem oder mehreren der Ansprüche 1 bis 3, dadurch gekennzeichnet, daß als polares (aliphatisches) Lösungsmittel mindestens eine Verbindung der folgenden Formeln
$C_qH_{2q+1}OH$ und $C_tH_{2t+1}O-(C_2H_4O)_uH$,
worin bedeuten: q eine Zahl von 1 bis 4, t die Zahl 1 oder 2 und u eine Zahl von 1 bis 3, verwendet wird.

5. Verfahren nach einem oder mehreren der Ansprüche 1 bis 4, dadurch gekennzeichnet, daß die Umsetzung mit dem Alkali-, Ammonium-oder Erdalkalisalz einer aliphatischen Carbonsäure mit 1 bis 6 C-Atomen in Gegenwart einer wirksamen, katalytischen Menge mindestens eines im Reaktionsgemisch löslichen Iodides durchgeführt wird.

6. Verfahren nach Anspruch 5, dadurch gekennzeichnet, daß als Iodid Natrium-oder Kaliumiodid verwendet wird.

7. Verfahren nach einem oder mehreren der Ansprüche 1 bis 6, dadurch gekennzeichnet, daß eine Verbindung der Formel II eingesetzt wird, in der k = Null ist.

8. Verfahren nach einem oder mehreren der Ansprüche 1 bis 7, dadurch gekennzeichnet, daß eine Verbindung der Formel I eingesetzt wird, in der A eine $-C_mH_{2m}$-Gruppe ist.

9. Verfahren nach einem oder mehreren der Ansprüche 1 bis 8, dadurch gekennzeichnet, daß eine Verbindung der Formel II eingesetzt wird, in der p eine Zahl von 1 bis 10 ist.

10. Verfahren nach einem oder mehreren der Ansprüche 1 bis 9, dadurch gekennzeichnet, daß die Umsetzung bei 100 bis 170 °C durchgeführt wird.

11. Verwendung der nach Anspruch 1 erzeugten Verbindungen der Formel I als Ausgangsstoff für die Herstellung von Verbindungen für die öl-und schmutzabweisende Ausrüstung von Textilien, Leder, Holz oder Papier.

Europäisches Patentamt

# EUROPÄISCHER RECHERCHENBERICHT

Nummer der Anmeldung

EP 86115436.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE - A1 - 3 021 447 (L'OREAL)<br>* Ansprüche 1,3; Beispiel 1 *<br>-- | 1,11 | C 07 C 43/13<br>C 07 C 43/11<br>C 07 C 43/12<br>C 07 C 143/74<br>D 06 M 13/18<br>D 06 M 13/40 |
| D,A | GB - A - 1 524 631 (KONISHIROKU PHOTO)<br>* Anspruch 1; Seiten 3-5 *<br>-- | 1 | |
| D,A | US - A - 3 470 258 (GIULIANA C. TESORO)<br>* Anspruch 1; Spalte 2 *<br>-- | 1 | |
| D,A | DE - A - 2 004 962 (UGINE KUHLMANN)<br>* Anspruch 1 *<br>-- | 1 | RECHERCHIERTE SACHGEBIETE (Int. Cl.4) |
| D,A | DE - A1 - 3 002 369 (MINNESOTA MINING)<br>* Ansprüche 1,2,8*<br>-- | 1,12 | C 07 C 43/00<br>C 07 C 143/00<br>D 06 M<br>C 07 C 41/00 |
| A | WO - A1 - 82/01 546 (CENTRE NATIONAL DE LA RECHERCHE)<br>* Anspruch 1 *<br>-- | 1 | |
| A | DE - A1 - 3 231 403 (DAINIPPON INK AND CHEMICALS)<br>* Ansprüche 1-5; Seite 24, Formula A,B *<br>-- | 1 | |

Der vorliegende Recherchenbericht wurde für alle Patentansprüche erstellt.

| Recherchenort | Abschlußdatum der Recherche | Prüfer |
|---|---|---|
| WIEN | 10-06-1987 | REIF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

EP 86115436.7

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | DE - B2 - 2 832 346 (BAYER)<br>* Anspruch 1 *<br>---- | 1 | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 10-06-1987 | REIF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82